# EUROPEAN PATENT SPECIFICATION

(11) **EP 2 114 517 B1**
(45) Date of publication and mention of the grant of the patent: **12.07.2017**
(21) Application number: 08728049.1
(22) Date of filing: 22.01.2008
(51) Int. Cl.: A61N 1/36, A61B 5/0482, A61B 5/0484

(54) **DEVICE FOR TREATING HUMAN VISION USING COMBINED OPTICAL AND ELECTRICAL STIMULATION**
VORRICHTUNG ZUR BEHANDLUNG DER MENSCHLICHEN SEHKRAFT MITHILFE KOMBINIERTER OPTISCHER UND ELEKTRISCHER STIMULATION
DISPOSITIF POUR TRAITER LA VISION HUMAINE EN UTILISANT UNE STIMULATION OPTIQUE ET ÉLECTRIQUE COMBINÉE

(30) Priority: 22.01.2007 US 886073 P
(43) Date of publication of application: 11.11.2009
(73) Proprietor: NovaVision Inc., Boca Raton FL 33487 (US)
(72) Inventor: PASCUAL-LEONE, Alvaro, Wayland, MA 01778 (US); PAUL, Patrick, J., Boca Raton, FL 33498 (US)
(74) Representative: Kransell & Wennborg KB
(86) International application number: PCT/US2008/051673
(87) International publication number: WO 2008/091876

(56) References cited:
- US-A- 6 061 593
- US-A1- 2002 087 201
- US-B2- 6 990 377

## Description

### Technical Field

The present invention relates to a therapeutic system for improving the sight of a subject. In particular, embodiments of the invention include using combinations of galvanic and optical stimulation.

### Background

Stimulating the vision system of human subjects suffering from vision impairment may improve visual performance of the subjects. For example, as documented in US Patent No 6,464,356, and US Published Patent Application No. 2005/0213033, presenting visual stimuli to areas of a human's visual system may allow improvement in the user's vision. NovaVision, of Boca Raton, Fl, produces VRT ™ (Visual Restoration Therapy) devices for effecting optical stimulation of defined locations of a patient's retina.

U.S. Patent Application Publication No. 2006/0283466, published December 21, 2006 describes a system and method for treating a retinal disease in a human that includes iteratively locating and defining a treatment area within a visual field, presenting visual stimuli to the treatment area at a specified location and definition, and recording changes in specified characteristics of the human's visual system.

U.S. Patent No 6,990,377, describes using an electrical pulse generator to stimulate neurons in conjunction with optical stimuli presented on a display.

U.S. Patent 5,522,864, teaches applying a current to the retina to effect treatment of ocular pathology.

It has been shown that external direct current stimulation of the visual cortex induces prolonged cortical excitability (Antal & al. Excitability changes induced in the human primary visual cortex by transcranial direct current stimulation: Direct electrophysiological evidence (Investigative Opthalmology & Visual Scieces Feb 2004 Volume 45 No 2))

### Summary of the Invention

The invention is defined in claim 1. Further aspects and preferred embodiments are defined in the dependent claims.

Aspects, embodiments and examples of the present disclosure which do not fall under the scope of the appended claims do not form part of the invention and are merely provided for illustrative purposes.

Furthermore, the methods presented in the present description are provided for illustrative purposes only and do not form part of the present invention. In accordance with an illustrative embodiment of the present invention, an integrated device is useful for improving the visual system of a human subject. The integrated device includes a computer that is in data communication with a display. The computer and display are adapted to present optical stimuli to a targeted region of an eye. The computer includes a timer for measuring a time relationship. A current source has at least one terminal for connection of at least one electrode assembly. A housing is adapted to contain the current source in combination with at least one of the display and the computer. The current source provides electrical stimulation to the subject's brain and the display provides optical stimulation to the subject's visual system in the time relationship.

In related embodiments, the time relationship may include providing the electrical stimulation prior to the optical stimulation, concurrent with the optical stimulation, or subsequent to the optical stimulation. The electrode assembly may include one or more electrodes, an electrode array, an orbital electrode or a sponge electrode. The electrode assembly may include at least one implanted electrode. The implanted electrode may be implanted subcutaneously in close proximity to the visual cortex. The implanted electrode may include a first induction coil for inductive coupling of energy from an external device to a stimulation electrode. The device may include an external actuator for actuating the implanted electrode without direct physical contact between the actuator and the electrode. The actuator may include a second induction coil. The electrode assembly may be adapted for transcranial stimulation targeting the occipital lobe.

Optionally or additionally, the housing may be adapted to be mounted on the head. Connection of one terminus of the electrode assembly to the device and another terminus of the electrode assembly to the subject may establish a direct current flow from the device to the subject The current source may provide continuous electrical power. The current source and computer may share a common housing. The current source and display may share a common housing. The current source, computer, and display may share a common housing. Optionally or additionally, the device may include a feedback module. The feedback module may include an EEG. The device may include at least one capacitor for isolating an EEG signal from a galvanic stimulation signal. The device may include at least one isolation transformer for isolating an EEG signal from a galvanic stimulation signal. The device may include a second current source. The first and second current sources may be electrically isolated. Also disclosed is a method for visual therapy includes applying an orbital electrode around the eye of a patient and applying a reference electrode to the patient. The orbital electrode and reference electrodes define a current path through a target visual structure of the nervous visual system of the patient. The method further includes identifying a desired zone for optical stimulation based on a visual field map of the patient, presenting an optical stimulus to the zone, measuring a response of the patient to the optical stimuli, and administering a galvanic current to the target visual structure via the current path. The administration of the galvanic current is synchronized with the optical stimulus.

Optionally or additionally, the method includes iterating the steps of identifying, presenting, measuring, and administering. Also disclosed is a method for visual therapy includes applying a cathodic electrode and an anodic electrode to the patient. The cathodic electrode and anodic electrode define a current path through a target visual structure of the nervous visual system of the patient. The anodic electrode is applied to be external and in contact with the skin of a patient. The method further includes identifying a desired zone for optical stimulation based on a visual field map of the patient, presenting an optical stimulus to the zone, measuring a response of the patient to the optical stimuli; and administering a galvanic current to the target visual structure via the current path. The administering of the galvanic current is synchronized with the optical stimulus.

Optionally or in addition, the method includes iterating the steps of identifying, presenting, measuring, and administering. An electrode used with the method may include a first induction coil and a second induction coil is used to transmit energy through the skin to thereby actuate the galvanic current flow.

### Brief Description of the Drawings

The foregoing features of the invention will be more readily understood by reference to the following detailed description, taken with reference to the accompanying drawings, in which:
Fig. 1 is a schematic diagram of a layout for an integrated electrical and optical stimulation device, in accordance with an embodiment of the invention;
Fig. 2 is a schematic view of patient head with an attached galvanic stimulation system.

### Detailed Description of Specific Embodiments

Definitions. As used in this description and the accompanying claims, the following terms shall have the meanings indicated, unless the context otherwise requires:
An "electrode assembly" shall mean any device or collection of devices for conduction of electrical energy from a source to a patient via one or more electrodes. The electrode assembly may include one or more individual or integrated reference and stimulation electrodes or electrode arrays.

An "electrode array" shall mean a collection of conductive terminals adapted for conduction via contact with a human subject, the terminals sharing a common conductor. The conductor is attachable to a source of electrical energy.

A "computer" shall mean any device or collection of local or networked devices, including a microcomputer or application-specific device, capable of performing the input, output, storage and computational functions of the various embodiments described herein.

In general, an integrated device delivers both galvanic (electrical) and optical stimulative therapy to improve the visual system of a human subject (a patient). Optical and electrical stimulation functionality may be combined in an integrated device. As a result, combined therapy may be delivered in a more convenient way, and the cost of delivering the therapy reduced. Another advantage of the integrated device is that galvanic therapy may be readily synchronized with the optical therapy and data associated with the delivery of or patient response to the therapy may be logged in a common file. As a result of using devices in accordance with embodiments of the invention, synergistic effects may be achieved. For example, galvanic treatment may be used to potentiate a physiological response to subsequent optical stimulation therapy. Embodiments also include safety and monitoring features to ensure patient compliance and prevent galvanic over-stimulation.

In illustrative embodiments, the device includes a computer and an illumination device, such as computer display (which may be a head-mounted display). The computer may actuate the display to present optical stimuli to the eye of the subject, including fixation stimuli in order to effect a course of visual restoration therapy. The computer may be used to record the patient's response to the optical and/or direct current stimulus. The computer may also control at least one current source for applying a current to the central nervous system (CNS) of the subject via electrodes, which may be affixed to the skin of the patient's head. In embodiments, the current, or a portion thereof, may flow through the patient's visual cortex, and may be a direct (DC) current. Another advantage is that parameters related to the current flow or optical presentations may be adaptively adjusted as a function of the subject's performance in responding to the stimuli. In various embodiments, a common housing may house the current source and the computer, or the current source, the computer and the display.

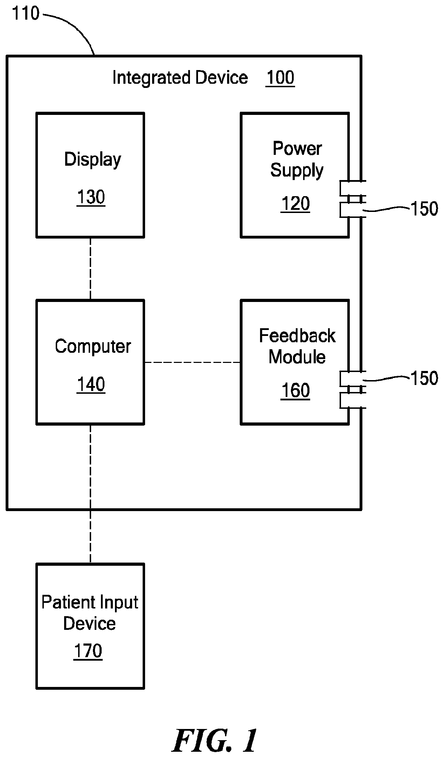

Fig. 1 schematically shows an integrated device 100 in accordance with an illustrative embodiment of the invention. The device 100 delivers both optical and direct current stimulation to a subj ect in order to achieve synergistic visual field restoration. The integrated device includes a housing 110, which contains at least one current source (i.e., an electrical power supply connectable to one or more circuits) 120 and at least one of a display 130, and/or a computer 140 in data communication with the display 130. Patient response device 170 (e.g., a mouse) allows a patient to input responses to optical stimulation. Each current source 120 may have a connector terminal for connecting an electrode assembly. Access to the connector may be provided through a port in the housing 110, or the current source 120 may protrude through a wall in the housing 110. The integrated device may be foldable and adjustable, as described in US Published Patent Application 2007/0171372, herein incorporated by reference in its entirety. The integrated device 100 of Fig. 1 also includes an optional feedback module 160 that makes measurements related to neuronal activity and allows adjustment of stimulative parameters of the device based on the measurements; an example of a feedback module is the EEG module described below.

Current maybe conducted from the current source(s) 120 to the head of the subject using a variety of electrode assembly types. The electrodes may be implanted into the brain, but for greater convenience and safety, and to lower the cost of the procedure, the electrodes may be attached to the skin. The skin-attached electrodes may be coated with a conducting gel to ensure acceptable conduction. Generally, one or more stimulation electrodes and one ore more reference electrodes are placed on the skin of the subject's head. The electrodes may be positioned to cause current flow to intersect with the patient's CNS, including areas related to vision. For example, a single stimulation and a single reference electrode may be used or an array of electrodes may be used. Combinations of single and reference electrodes are also possible; e.g., a stimulation electrode array and a single reference electrode or vice versa. A pad or sponge electrode (e.g., impregnated with saline) may be also be used. U.S. Patent 6,263,226 to Axelgaard issued July 17, 2001 describes a sponge electrode. A pad or sponge electrode may have the advantage of applying current diffusely across a large area of skin. However, a large area may also be covered using a spaced-apart array of electrodes. The electrode array may be integrated into a soft, elastic cap that applies an elastic force to hold an array of spaced-apart electrodes against the subject's head. In a particular configuration, an anodal electrode is applied in the immediate region of the occipital lobe, and a reference electrode (cathode) applied elsewhere, in a manner such that the direct current stimulation will flow at least in part through the visual cortex. In addition, US Patents 4,092,985; 3,977,392; 3,376,870; 2,943,627; 6,208,902; 7317,947 and RE31,454 discloses electrodes that may be used with embodiments of the invention. Additionally a tripolar, concentric-ring, or double-concentric ring electrode arrangement may be used.

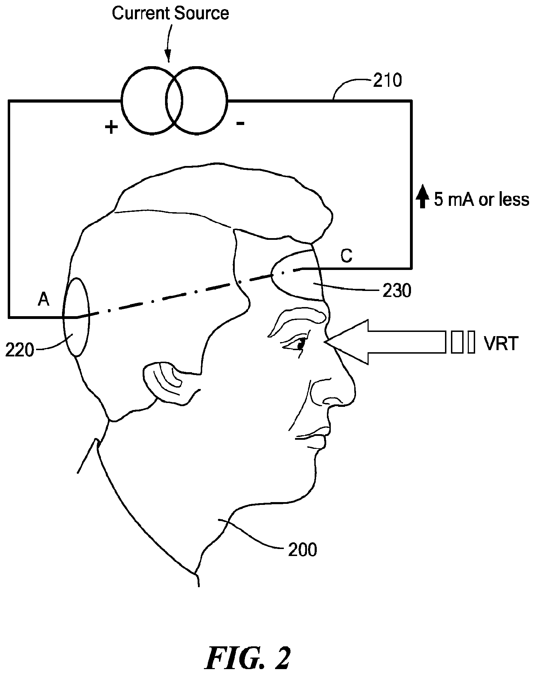

Fig. 2 schematically shows an embodiment of the invention in which a patient 200 is connected to a galvanic stimulation circuit 210 via an anodic electrode 220 and a cathodic electrode 230. The electrodes (220,230) are positioned to direct electron flow through the visual cortex of the patient 200. A current source is used to deliver a DC current through the visual cortex. The current is limited to 5 mA or less. Optical stimuli (e.g., VRT) are presented to the patient while current is flowing through the visual cortex of the patient 200.

Generally electrodes may be placed in close proximity to at least a portion of the visual system (e.g. the visual cortex) to establish a current path through the visual system portion. Placement of the electrodes may be difficult for an individual user or lay caregiver to perform. For example, hair may interfere with establishing adequate electrical contact, electrodes may drip saline and result in uncontrolled stray currents on the skin, and the electrodes may be difficult to properly and consistently position on the scalp. An occipital lobe electrode may be especially difficult to place due to the need for location at the back of the head. Accordingly, in an embodiment of the invention, electrodes, including the reference electrode may be implanted subcutaneously, e.g., beneath the scalp of the patient.For example, a surgeon may implant two electrodes under the scalp. The electrodes may be powered either via a transdermal subminiature connector or magnetically, via a power-coupling wand which the patient or caregiver positions above the electrodes. Methods for implanting electrodes and coupling them to external power sources are known in the art; for example, US Patents Nos. 7,010,351, 75,540,736, and 6,208,902 discloses a variety of such methods. In an embodiment at least one stimulation electrode is implanted subcutaneously and above the skull along with a first induction coil and associated circuitry. An external device that includes a second induction coil connected to a power source is held in proximity to the first induction coil to transfer energy through the skin and induce a current flow.

The anode may be located just above the occipital lobe, and the reference electrode (cathode) in a location which would favor a current path through the visual cortex. As a result, a reliable and consistent DC current path is established. In a variation, only a cortical stimulation electrode is implanted under the scalp, and in close proximity to the occipital lobe. The cortical stimulation electrode is accessible via a transdermal subminiature connector. One or more reference electrodes are positioned externally (on the skin), and may be applied to the forehead during each session. The reference electrodes are positioned to establish a current path through the visual cortex.

In another embodiment, an electrode may be placed around the subject's eye to stimulate and thereby treat the retina of a subject with a retinal deficit. The electrode may be an annular electrode applied to the orbit of the eye (i.e., an orbital electrode), that allows the patient to see through the electrode. Use of such an electrode will allow the subject to receive optical stimuli via a corneal pathway while wearing the electrode. A reference electrode may be placed on the skin on the occiput (i.e., an occipital electrode) so that stimulation current may flow between the electrodes and through the subject's retina and ocular structures, along the optic nerve, though the occipital lobe, and to the reference electrode.

The current used for the purpose of therapy may be imperceptible to the patient. Consequently, the patient cannot determine if the current is actually flowing. The computer may continuously monitor the output of each current source, and may notify the patient that the electrodes have been properly placed and that current is flowing. During therapy, the device may notify the subject, if, for any reason the current of one or more of the current sources is interrupted. In response, the subject or caregiver may then suspend therapy until the current flow is resumed. For example, if the computer detects an elevated contact resistance with the skin indicative of a dislodged electrode, a warning message or sound may be presented via the display or a computer associated loudspeaker.

Similarly, if the patient pauses the therapy, both galvanic and visual therapies can be suspended. The subject may thereby be allowed to disconnect from the device and return at a later time to resume a sequence of therapy.

In order to reliably deliver a safe, effective, and reproducible amount of charge to the subject, the current source may be a constant current source. The constant current source may comprise, for example a battery, a rechargeable battery, or an isolated power supply together with necessary circuitry to produce a constant current that is independent of the load applied to the current source. The current produced by the constant current source is independent from a connected load. For example, a constant current source may produce a current with an intensity of 500µA ± 10% (standard deviation) in the presence of either a 5,000 Ohm or a 10,000 Ohm resistor. Alternately, the constant current source may produce a current of 1 mA with ±10 %(standard deviation) in intensity when the load is varied from 10,000 Ohms to 100,000 Ohms. The constant current source intensity may be programmable and may be controlled by the computer 140 and associated software. A representative embodiment has available settings from between 1 and 5 mA, adjustable in 1 µA steps, and may effectively handle loads (i.e., inter-electrode resistance) of between 5000 Ohm. to 100,000 Ohm. The computer 140 may include a safety feature that ensures that the voltage resulting from the direct current flowing through the inter-electrode resistance never exceeds a safe and comfortable level of 20 VDC or less.

For ease of connection, each current source may have one or more terminal connectors (e.g., that donate or accept a pin or banana-plug) that are accessible through the housing. The current source may deliver a current via the electrode assembly that is in the range of 5 µA to 10 mA, and more preferably between 10 µA and 5 mA. The computer may control one or more electrical parameters. For example, the current may be turned on, turned off, the intensity/amplitude adjusted or voltage limited, and the electrical polarity switched in response to computer command. The computer, based on a therapy profile specific to the subject, may set the intensity of current or other electrical output parameter. The computer may also log the current level at one or more times during therapy for later review; for example, a log entry may be created every second. The logged information may be associated with files holding other session information such as the user biographic profile, therapy history, subject performance information, and visual field maps. The computer may include a timer for synchronizing the presentation of optical and galvanic stimuli. The synchronization may include measuring a predetermined time relationship between application of the optical and galvanic stimuli. Direct current application may be administered before, during, or after optical stimulation (including single stimulation events and sequences or courses of optical stimulation therapy). The timing of the direct current and optical stimulation may be offset by a predetermined amount. For example, direct current stimulation may precede the presentation of an optical stimulation by a period of between 3 seconds and 30 minutes. The current may also be applied in a continuous manner throughout a session of optical stimulation therapy for time periods lasting from a few minutes to several hours. Alternately, direct current stimulation may be applied after the optical stimulation, thereby synergistically enhancing (i.e., consolidating) the therapeutic effects of the previous visual stimulation. The computer may also record, via an input device, the subject's perception or lack of perception of the electrical stimulation.

During the course of the visual stimulation therapy, which may last several months, the location of the visual stimuli on the display, and the galvanic stimulation may be adjusted to reflect changes in the patient response to the stimuli in specific zones of the patient visual field. Adjustments of the galvanic stimulation during therapy may include changes amperage (intensity), current polarity, duration of current flow, and electrode locations.

In another embodiment, multiple current sources may also be integrated into the device. Using multiple current sources facilitates stimulation of various parts of the CNS with different electronic regimes (e.g. current, polarity or timings). For example, in a therapeutic regime that includes stimulation of independent CNS regions, a first pair of electrodes is applied to occipital lobe and delivers a current having a first intensity, while a second pair, connected to a second current source stimulates the optic nerve with a second intensity.

Accordingly, the respective current sources may be controlled independently. For example, the computer may independently trigger switching the current on and off, alter current levels, etc. One or more current source may be electrically isolated (i.e., floating) with respect to other current sources. As a result, multiple independent reference electrodes may be used effectively. Multiple current sources, electrically floating with respect to each other, are useful in better managing stray currents between electrodes applied to separate regions of the CNS

For safety purposes, each current source may also be electrically isolated with respect to the electrical ground on the device. Each current source may have a pair of terminals located on the housing of the device for interfacing with reference and stimulation electrode assemblies. The computer may also log data regarding operation of the multiple current sources.

The galvanic stimulation may comprise application of a relatively small current, e.g., a direct current of between 10 µA and 5 mA. In specific embodiments, the galvanic stimulation may be applied for a long period, e.g., up to 3 hours.

In order to preclude the possibility of CNS damage due to overstimulation, the duration and intensity of galvanic stimulation should be limited to a safe level. Safe stimulation protocols have to be developed which allow an optimum galvanic stimulation while enforcing rigorous subject safety. Agnew and McCreery (1987) (cf. Nitsche et al) give indications of safety guidelines. The computer of the device may be programmed with maximum subject exposure. Exposure may be measured in Coulomb per day. Using the formula Q = I x t, one Coulomb is defined as the electrical charge corresponding to 1 Ampere flowing for 1 second.

The computer 140 may be programmed with specific current limits and Coulomb limits for subject exposure over established period of time (e.g., an hour or day). Maximum exposure thresholds may be associated with a given subject via use of a subject identifier, e.g., a username, smart-card, etc.

Coulomb exposure can be tracked for each subject. Once a maximum Coulomb exposure threshold is reached, the therapy could be terminated or optical stimulation (e.g., VRT) could continue without the galvanic stimulation. Resumption of galvanic stimulation may then be permitted after an established recovery period measured by the computer.

Implementation of the above safety features may encourage use of the device in a home, and in the absence of dedicated health professionals, thus dramatically lowering the cost of therapy.

An embodiment of the device that further enhances the safety of the application of galvanic stimulation to the visual system of a patient incorporates includes a feedback module 160 that continuously monitor an EEG (electroencephalogram) of the patient during the galvanic stimulation. This EEG may be recorded using the same electrodes used for the galvanic stimulation, or a separate system of electrodes. If a separated set of electrodes are used, the integrated device may have connection ports to accommodate leads from these electrodes. The EEG may be processed using a real-time algorithm which repeatedly analyzes brain activity and adjusts the amount and characteristics of the direct current stimulation. A common housing may house the direct current source, the EEG circuitry, the computer and all necessary software required to monitor the EEG in real-time and correspondingly adapt the direct current stimulation. Such an embodiment may include the display, or the display can be a separate Head Mounted Device (HMD), which may incorporate within its structure, direct current stimulation electrodes and/or EEG electrodes. The direct current may be isolated from the EEG circuitry using capacitors. In addition, the device may include circuitry to filter and process the EEG signal to remove the galvanic stimulation signal and other noise; using, for example, a digital signal processor (DSP). This circuitry may include at least one isolation transformer. The EEG feedback module may incorporate principles or hardware of known EEG monitoring systems; for example, BIS Technology, which is commercialized by Aspect Medical Systems, Inc or Norwood, MA (www.aspectmedical.com)*.*

The display may be any suitable computer-driven display; for example, a CRT, LCD, plasma, SED, or OLED. The display may be a desktop-type display, adjustably mounted, or may be a head mounted display (e.g., goggles or a helmet). If a head mounted display is used, the current source and computer may be separately housed to decrease the weight and bulk of the head mounted device portion. The head mounted display may be in wired or wireless communication with the computer. Optionally, stimulation and EEG electrodes may be integrated with the head mounted display.

Embodiments of the integrated system described above, patients may be used to deliver VRT according to a variety of optical stimulation, testing and feedback regimes. The VRT therapy typically includes creating a visual field map, targeting particular zones of the visual field with optical stimuli, measuring the resulting changes in the visual field, and redefining the zones based on the revised measurement. Optical stimulation regimes may be supplemented with electrical stimulation as described above. These regimes include those described in the referenced listed in table below.

**Table 1.**

| |
|---|
| U.S. Patent No. 6,464,356 |
| U.S. Published application No. 2005-0213033 |
| U.S. Published application No. 2006-0283466 |
| U.S. Published application No. 2007-0038142 |
| U.S. Published application No. 2007-0171372 |
| U.S. Published application No. 2007-0182928 |
| U.S. Published application No. 2007-0216865 |
| U.S. Published application No. 2008-0013047 |
| U.S. Patent application Serial No. 11/782,379, filed 7/24/2007 |
| U.S. Patent application Serial No. 11/881,140, filed 7/25/2007 |

A variety of schemes may be used to synchronize the galvanic therapy with the VRT. For example, galvanic stimulation may be applied before, during or after a VRT session, and may be applied continuously or in a series of pulses. Combination of the aforementioned electrical and optical stimulation may provide synergistic therapeutic effects. In other words, greater degrees and rates of improvement may be possible using the combined stimulation approach than may be obtained by either individually. Without wanting to be bound by the mechanism, the synergistic effect may involve increased cortical excitability due to galvanic stimulation. As a result, embodiments described herein may be effective in the treatment of vision loss including vision loss resulting from neurological damage of visual cortex or optic nerve. Such neurological damage may result from stroke, traumatic brain injury, and age related macular degeneration, among others.

In alternative examples the disclosed methods for visual field stimulation may be implemented as a computer program product for use with a computer system. Such implementations may include a series of computer instructions fixed either on a tangible medium, such as a computer readable medium (*e.g.*, a diskette, CD-ROM, ROM, or fixed disk) or transmittable to a computer system, via a modem or other interface device, such as a communications adapter connected to a network over a medium. The medium may be either a tangible medium (*e.g*., optical or analog communications lines) or a medium implemented with wireless techniques (*e.g.*, microwave, infrared or other transmission techniques). The series of computer instructions embodies all or part of the functionality previously described herein with respect to the system. Those skilled in the art should appreciate that such computer instructions can be written in a number of programming languages for use with many computer architectures or operating systems.

Furthermore, such instructions may be stored in any memory device, such as semiconductor, magnetic, optical or other memory devices, and may be transmitted using any communications technology, such as optical, infrared, microwave, or other transmission technologies. It is expected that such a computer program product may be distributed as a removable medium with accompanying printed or electronic documentation (*e.g.*, shrink wrapped software), preloaded with a computer system (*e.g.,* on system ROM or fixed disk), or distributed from a server or electronic bulletin board over the network (*e.g*., the Internet or World Wide Web). Of course, some embodiments of the invention may be implemented as a combination of both software (*e.g.*, a computer program product) and hardware. Still other embodiments of the invention are implemented as entirely hardware, or entirely software (*e.g.,* a computer program product).

The described embodiments of the invention are intended to be merely exemplary and numerous variations and modifications will be apparent to those skilled in the art. All such variations and modifications are intended to be within the scope of the present invention as defined in the appended claims.

## Claims

1. An integrated device (100) for improving the visual system of a human subject (200) having an eye, a head, and a skull, the device comprising:
- a computer (140) in data communication with a display (130), the computer (140) and display (130) adapted to present optical stimuli to a targeted region of an eye, the computer (140) including a timer for measuring a time relationship;
- a current source (120) having at least one connector for connection of at least one electrode assembly (220, 230),
- wherein the current source (120) provides electrical stimulation to the subject's (200) brain and the display (130) provides optical stimulation to the subject's (200) visual system in the time relationship,
wherein the time relationship includes providing the electrical stimulation prior to, concurrent with or subsequent to the optical stimulation,
**characterized by** a housing (110) adapted to contain the current source in combination with at least one of the display and the computer,
wherein the computer (140) is adapted to control the current source (120) such that specific current limits and specific coulomb limits for exposure of the human subject (200) over an established period of time are not exceeded.

2. The device according to claim 1, wherein the electrode assembly (220, 230) comprises one of an electrode, an electrode array, an orbital electrode and a sponge electrode.

3. The device according to claim 1, wherein the electrode assembly (220, 230) comprises at least one implanted electrode.

4. The device according to claim 3, wherein the implanted electrode is implanted subcutaneously in close proximity to the visual cortex.

5. The device according to claim 4, wherein the implanted electrode includes a first induction coil for inductive coupling of energy from an external device to the stimulation electrode.

6. The device according to any of claims 3 or 4, further comprising an external actuator for inductively powering and actuating the implanted electrode without direct physical contact between the actuator and the electrode, wherein the actuator in particular includes a second induction coil.

7. The device according to claim 1, wherein the electrode assembly (220, 230) is adapted for transcranial stimulation targeting the occipital lobe with anodal current.

8. The device according to claim 7, wherein the housing (110) is adapted to be mounted on the head.

9. The device according to claim 1, wherein connection of one terminus of the electrode assembly to the device and another terminus of the electrode assembly to the subject establishes a direct current flow from the device to the subject.

10. The device according to claim 1, wherein the current source (120) and computer (140) share a common housing and/or wherein the current source (120) and display (130) share a common housing.

11. The device according to claim 1, further comprising a feedback module (160), wherein the feedback module in particular comprises an EEG module.

12. The device according to claim 11, further comprising
- at least one capacitor for isolating an EEG signal from a galvanic stimulation signal; or
- at least one isolation transformer for isolating the EEG module from a galvanic stimulation signal.

13. The device according to claim 1, further comprising a second current source, wherein the first and second current sources are in particular electrically isolated.

## Patentansprüche

1. Integrierte Vorrichtung (100) zur Verbesserung des visuellen Systems einer menschlichen Person (200) mit einem Auge, einem Kopf und einem Schädel, wobei die Vorrichtung aufweist:
- einen Computer (140) in Datenkommunikation mit einer Anzeige (130), wobei der Computer (140) und die Anzeige (130) dazu ausgelegt sind, einem Zielbereich eines Auges optische Stimuli darzureichen, wobei der Computer (140) einen Zeitgeber zum Messen eines zeitlichen Zusammengangs aufweist;
- eine Stromquelle (120) mit mindestens einem Verbinder zur Verbindung mindestens einer Elektrodenanordnung (220, 230),
- wobei die Stromquelle (120) eine elektrische Stimulation für das Gehirn der Person (200) bereitstellt und die Anzeige (130) eine optische Stimulation für das visuelle System (200) der Person in dem zeitlichen Zusammenhang bereitstellt,
elektrischen Stimulation vor, gleichzeitig mit, oder nach der optischen Stimulation aufweist,
**gekennzeichnet durch** ein Gehäuse (110), das die Stromquelle in Kombination mit mindestens eines von der Anzeige und dem Computer enthalten kann,
wobei der Computer (140) dazu ausgelegt ist, die Stromquelle (120) so zu steuern, dass spezifische Stromgrenzen und spezifische Coulomb-Grenzwerte für die Exposition der menschlichen Person (200) über einen festgelegten Zeitraum nicht überschritten werden.

2. Vorrichtung nach Anspruch 1, wobei die Elektrodenanordnung (220, 230) eines von einer Elektrode, einem Elektrodenarray, einer Orbitalelektrode, und einer Schwammelektrode aufweist.

3. Vorrichtung nach Anspruch 1, wobei die Elektrodenanordnung (220, 230) mindestens eine implantierte Elektrode aufweist.

4. Vorrichtung nach Anspruch 3, wobei die implantierte Elektrode subkutan in unmittelbarer Nähe des visuellen Kortex implantiert ist.

5. Vorrichtung nach Anspruch 4, wobei die implantierte Elektrode eine erste Induktionsspule zur induktiven Kopplung von Energie aus einer externen Vorrichtung zu der Stimulationselektrode aufweist.

6. Vorrichtung nach einem der Ansprüche 3 oder 4, ferner aufweisend einen externen Aktuator zur induktiven Versorgung und Betätigung der implantierten Elektrode ohne direkten physikalischen Kontakt zwischen dem Aktuator und der Elektrode, wobei der Aktuator insbesondere eine zweite Induktionsspule aufweist.

7. Vorrichtung nach einem der Ansprüche 1 bis 6, wobei die Elektrodenanordnung (220, 230) für eine transkranielle, auf den Okzipitallappen abzielende Stimulation mit anodalem Strom ausgelegt ist.

8. Vorrichtung nach Anspruch 7, wobei das Gehäuse (110) auf dem Kopf montierbar ist.

9. Vorrichtung nach einem der Ansprüche 1 bis 9, wobei die Verbindung eines Endpunktes der Elektrodenanordnung mit der Vorrichtung und eines anderen Endpunktes der Elektrodenanordnung mit der Person einen Gleichstromfluss von der Vorrichtung zu der Person herstellt.

10. Vorrichtung nach einem der Ansprüche 1 bis 10, wobei die Stromquelle (120) und der Computer (140) sich ein gemeinsames Gehäuse teilen und/oder wobei die Stromquelle (120) und die Anzeige (130) sich ein gemeinsames Gehäuse teilen.

11. Vorrichtung nach einem der Ansprüche 1 bis 11, ferner aufweisend ein Rückkopplungsmodul (160), wobei das Rückkopplungsmodul insbesondere ein EEG-Modul aufweist.

12. Vorrichtung nach Anspruch 11, ferner aufweisend
- mindestens einen Kondensator zur Trennung eines EEG-Signals von einem galvanischen Stimulationssignal;
Oder
- mindestens einen Trenntransformator zur Trennung des EEG-Moduls von einem galvanischen Stimulationssignal.

13. Vorrichtung nach Anspruch 1, ferner aufweisend eine zweite Stromquelle, wobei die erste und die zweite Stromquelle insbesondere elektrisch getrennt sind.

## Revendications

1. Dispositif intégré (100) pour l'amélioration du système visuel d'un sujet humain (200) doté d'un oeil, d'une tête et d'un crâne, ce dispositif comprenant :
- un ordinateur (140) en communication de données avec un afficheur (130), l'ordinateur (140) et l'afficheur (130) étant aptes à présenter des stimuli optiques à une région ciblée d'un oeil, l'ordinateur (140) comprenant un temporisateur pour mesurer une relation temporelle ;
- une source de courant (120) comportant au moins un connecteur pour la connexion d'un ensemble d'électrodes (220, 230),
- la source de courant (120) fournissant une stimulation électrique au cerveau du sujet (200) et l'afficheur (130) fournissant une stimulation électrique au système visuel du sujet (200) en relation temporelle,
- la relation temporelle incluant la fourniture de la stimulation électrique avant, concurremment ou subséquemment à la stimulation optique,
**caractérisé par** un boîtier (110) apte à contenir la source de courant en combinaison avec au moins un élément parmi l'afficheur et l'ordinateur,
l'ordinateur (140) étant apte à commander la source de courant (120) de manière à ne pas dépasser des limites de courant spécifiques et des limites de Coulomb spécifiques pour l'exposition du sujet humain (200) pendant une durée établie.

2. Dispositif selon la revendication 1, dans lequel l'ensemble d'électrodes (220, 230) comprend un élément parmi une électrode, un réseau d'électrodes, une électrode orbitale et une électrode à éponge.

3. Dispositif selon la revendication 1, dans lequel
l'ensemble d'électrodes (220, 230) comprend au moins une électrode implantée.

4. Dispositif selon la revendication 3, dans lequel
électrode implantée est implantée sous la peau à proximité immédiate du cortex visuel.

5. Dispositif selon la revendication 4, dans lequel l'électrode implantée comprend une première bobine d'induction pour le couplage inductif d'énergie en provenance d'un dispositif externe vers l'électrode de stimulation.

6. Dispositif selon l'une quelconque des revendications 3 ou 4, comprenant en outre un actionneur externe pour alimenter et actionner de manière inductive l'électrode implantée sans contact physique direct entre l'actionneur et l'électrode, l'actionneur comprenant particulier une seconde bobine d'induction.

7. Dispositif selon la revendication 1, dans lequel l'ensemble d'électrodes (220, 230) est apte à effectuer une stimulation transcrânienne ciblant le lobe occipital avec du courant anodique.

8. Dispositif selon la revendication 7, dans lequel le boîtier (110) est apte à être monté sur la tête.

9. Dispositif selon la revendication 1, dans lequel la connexion d'un terminus de l'ensemble d'électrodes au dispositif et d'un autre terminus de l'ensemble d'électrodes au sujet établit un flux de courant direct du dispositif au sujet.

10. Dispositif selon la revendication 1, dans lequel la source de courant (120) et l'ordinateur (140) partagent un boîtier commun et/ou dans lequel la source de courant (120) et l'afficheur (130) partagent un boîtier commun.

11. Dispositif selon la revendication 1, comprenant en outre un module de retour (160), le module de retour comprenant en particulier un module EEG.

12. Dispositif selon la revendication 11, comprenant en outre
- au moins un condensateur pour isoler un signal EEG d'un signal de stimulation galvanique, Ou
- au moins un transformateur d'isolation pour isoler le module EEG d'un signal de stimulation galvanique.

13. Dispositif selon la revendication 1, comprenant en outre une seconde source de courant, les première et seconde sources de courant étant en particulier isolées électriquement.
